# EUROPEAN PATENT SPECIFICATION

(11) **EP 1 026 259 B1**
(45) Date of publication and mention of the grant of the patent: **27.11.2002**
(21) Application number: 00102619.4
(22) Date of filing: 08.02.2000
(51) Int. Cl.: C12Q 1/68

(54) **PROCESS FOR PREPARING A DNA CHIP**
VERFAHREN ZUR HERSTELLUNG EINES DNS CHIP
PROCEDE DE PREPARATION D'UN CHIP D'ADN

(30) Priority: 08.02.1999 JP 3042999
(43) Date of publication of application: 09.08.2000
(73) Proprietor: FUJI PHOTO FILM CO., LTD., Kanagawa-ken, 250-0123 (JP)
(72) Inventor: Kuhara, Satoru, Fukuoka-shi, Fukuoka, 811-1355 (JP); Tashiro, Kosuke, Fukuoka-shi, Fukuoka, 813-0043 (JP); Muta, Shigeru, Fukuoka-shi, Fukuoka, 812-0063 (JP); Tsuchiya, Toru, Ashigarakami-gun, Kanagawa-ken 258-8538 (JP); Hakamata, Masashi, Ashigarakami-gun, Kanagawa-ken 258-8538 (JP)
(74) Representative: Weisert, Annekäte, Dipl.-Ing. Dr.-Ing.

(56) References cited:
- WO-A-95/35505
- WO-A-98/39481
- SHALON D ET AL: "A DNA MICROARRAY SYSTEM FOR ANALYZING COMPLEX DNA SAMPLES USING TWO-COLOR FLUORESCENT PROBE HYBRIDIZATION" GENOME RESEARCH,US,COLD SPRING HARBOR LABORATORY PRESS, vol. 6, no. 7, 1 July 1996 (1996-07-01), pages 639-645, XP000597095 ISSN: 1088-9051
- KHRAPKO K R ET AL: "HYBRIDIZATION OF DNA WITH OLIGONUCLEOTIDES IMMOBILIZED IN GEL: A CONVENIENT METHOD FOR DETECTING SINGLE BASE SUBSTITUTIONS" MOLECULAR BIOLOGY,XX,XX, vol. 25, no. 3, 1 May 1991 (1991-05-01), pages 581-591, XP000576468 ISSN: 0026-8933
- SCHENA M ET AL: "PARALLEL HUMAN GENOME ANALYSIS: MICROARRAY-BASED EXPRESSION MONITORING OF 1000 GENES" PROCEEDINGS OF THE NATIONAL ACADEMY OF SCIENCES OF USA,US,NATIONAL ACADEMY OF SCIENCE. WASHINGTON, vol. 93, no. 20, 1 October 1996 (1996-10-01), pages 10614-10619, XP002022507 ISSN: 0027-8424
- WATTS H J ET AL: "REAL-TIME DETECTION AND QUANTIFICATION OF DNA HYDRIDAZATION BY AN OPTICAL BIOSENSOR" ANALYTICAL CHEMISTRY,US,AMERICAN CHEMICAL SOCIETY. COLUMBUS, vol. 67, no. 23, 1 December 1995 (1995-12-01), pages 4283-4289, XP000540115 ISSN: 0003-2700
- SCHENA M. ET AL.,: "Microarrays:biotechnology's discovery platform for functional genomics" TIBTECH , vol. 16, - July 1998 (1998-07) pages 301-306, XP002139838

## Description

### FIELD OF THE INVENTION

This invention relates to a process for preparing a DNA chip favorably employable for detecting a DNA fragment complementary to oligonucleotide or polynucleotide attached to the DNA chip and its preparation.

### BACKGROUND OF THE INVENTION

In the gene analysis in the fields of biochemistry and clinical test, the detection of a DNA fragment having a specific base sequence is performed by way of a hybridization method, particularly Southern hybridization method (i.e., Southern blotting method). Southern hybridization is performed by the steps of cleaving a DNA to be examined (i.e., sample DNA) by the use of a restriction enzyme to give its fragments; separating the DNA fragments having different molecular sizes by electrophoresis on agarose gel or polyacrylamide gel; subjecting the separated DNA fragment to treatment for giving a single stranded DNA fragment; fixing the single stranded DNA fragment onto a polyamide filter or a nitrocellulose filter; hybridizing the fixed single stranded DNA with a probe DNA (i.e., a single stranded DNA which is complementary to the fixed single stranded DNA and which is labelled with RI (i.e., radioactive isotope); washing the filter; and subjecting the filter to autoradiography for visualizing the hybridized DNA fragment on the filter.

The conventional methods using radioisotope label such as Southern hybridization method have a disadvantageous feature that they need radioisotopes which should be treated with extremely high care. Moreover, the autoradiographic process requires a long period of time such as 24 hours or longer. In the case that only a small amount of sample DNA is available, the autoradiographic process requires a longer period of time and it does not give clear separated bands.

A Southern hybridization method in which a fluorescent label is used in place of the radioisotope label and the detection is performed by fluorometry is also known. Accordingly, a DNA chip comprising a substrate (i.e., solid carrier) such as a slide glass or a silicone plate and a great number of oligonucleotides or polynucleotides fixed onto the substrate are now commercially available for the use in the fluorescence detection systems.

At present, two methods are known for preparing a DNA chip having a solid carrier and oligonucleotide or polynucleotide fixed onto the carrier. One preparation method comprises preparing oligonucleotide or polynucleotide step by step on the carrier. This method is named "on-chip method". A typical on-chip method is described in Foder, S.P.A., Science, 251, page 767 (1991).

Another preparation method comprises attaching a separately prepared oligonucleotide or polynucleotide onto a solid carrier. Various methods are known for various oligonucleotides and polynucleotides.

In the case that the oligonucleotide or polynucleotide is cDNA fragment (i.e., complementary DNA fragment which is synthesized using mRNA as mold) or PCR product (which is a DNA fragment prepared by multiplying cDNA by PCR method), an aqueous solution of the prepared DNA fragment is spotted onto a solid carrier having a polycationic coat in a DNA chip-preparing device to attach the DNA fragment to the carrier via electrostatic bonding, and then blocking a free surface of the polycationic coat.

In the case that the oligonucleotide is synthetically prepared and has a functional group, an aqueous solution of the synthetic oligonucleotide is spotted onto an activated solid carrier to produce covalent bonding between the oligonucleotide and the carrier surface. See Lamture, J.B., et al., Nucl. Acids Res., 22, 2121-2125, 1994, and Guo, Z., et al., Nucl. Acids Res., 22, 5456-5465, 1994. Generally, the oligonucleotide is covalently bonded to the surface activated carrier via a spacer or a cross-linker. Also known is a process comprising the steps of aligning small polyacrylamide gels on a glass plate and fixing synthethized oligonucleotides onto the glass plate by making a covalent bond between the polyacrylamide and the oligonucleotide (Yershov, G., et al., Proc. Natl. Acad. Sci. USA, 94, 4913(1996)). Sosnowski, R.G., et al., Proc. Natl. Acad. Sci. USA, 94, 1119-1123 (1997) discloses a process comprising the steps of an array of microelectrodes on a silica chip, forming on the microelectrode a streptoavidin-comprising agarose layer, and attaching biotin-modified DNA fragment to the agarose layer by positively charging the agarose layer. Schena, M., et al., Proc. Natl. Acadl Sci. USA, 93, 10614-10619 (1996) teaches a process comprising the steps of preparing a suspension of an amino group-modified PCR product in SSC (i.e., standard sodium chloride-citric acid buffer solution), spotting the suspension onto a slide glass, incubating the spotted glass slide, treating the incubated slide glass with sodium borohydride, and heating thus treated slide glass.

As is explained above, most of the known methods of fixing a separately prepared DNA fragment onto a solid carrier utilize an electrostatic bonding or a covalent bonding such as described above.

In any DNA chip having a separately prepared DNA fragment on its solid carrier, the DNA fragment should be firmly fixed onto the carrier, so as to perform smoothly the hybridization between the fixed DNA fragment and a sample DNA fragment complementary to the fixed DNA fragment.

### SUMMARY OF THE INVENTION

It is an object of the present invention to provide a process for preparing a DNA chip having oligonucleotide or polynucleotide which is firmly fixed onto a solid carrier.

It is another object of the invention to provide a method for fixing oligonucleotide or polynucleotide onto a solid carrier utilizing a relatively simple means.

It is a further object of the invention to provide a process for detecting a DNA fragment complementary to oligonucleotide or polynucleotide fixed onto a DNA chip.

The present invention resides in a process for preparing a DNA chip comprising a solid carrier and oligonucleotide or polynucleotide which is fixed to the carrier, preferably at its one end portion, in the presence of a hydrophilic polymer.

The invention also resides in a method of fixing an oligonucleotide or polynucleotide to a solid carrier at its one end portion which comprises spotting an aqueous solution containing the oligonucleotide or polynucleotide and a hydrophilic polymer onto the carrier.

### BRIEF DESCRIPTION OF DRAWINGS

Fig. 1 schematically shows a process for detecting a DNA fragment complementary to oligonucleotide or polynucleotide fixed to a DNA chip of the present invention.

Fig. 2 is an enlarged view of the DNA chip on which fluorescence indicator-labelled complementary DNA fragments are attached to the oligonucleotide by hybridization.

### DETAILED DESCRIPTION OF THE INVENTION

The fixation of oligonucleotide or polynucleotide on a solid carrier according to the invention is accomplished by means of a hydrophilic polymer. Detailed mechanism of the fixation by the hydrophilic polymer is not known yet. It is assumed, however, that the hydrophilic polymer assists the known electrostatic bonding between a solid carrier and the terminal site of oligonucleotide or polynucleotide. Further, a high viscosity of a hydrophilic polymer may be effective for forming firm fixation of oligonucleotide or polynucleotide onto a solid carrier.

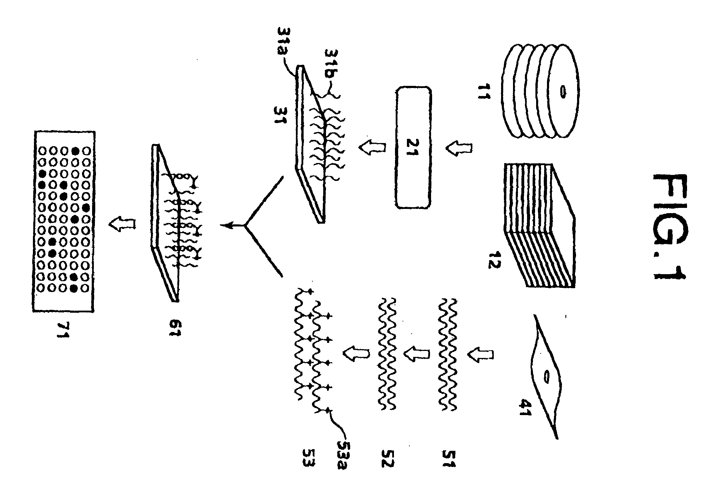

Fig. 1 illustrates a flow chart indicating the preparation of a DNA chip and a process for detecting a DNA fragment complementary to oligonucleotide or polynucleotide fixed onto the DNA chip. This flow chart is shown in the known publication, Protein, Nucleotide, Enzyme, vol. 43, NO. 13, 1998.

According to the database 11 concerning genome sequence, cDNA sequence, or EST (i.e., cDNA fragment of 200 to 300 bp (bp: base pair) from 3'-terminal), or from clones 12, oligonucleotide or polynucleotide 21 (e.g., cDNA, EST, or oligo DNA) is produced by PCR multiplication process or chemical synthesis. The oligonucleotide or polynucleotide 21 is fixed onto a solid carrier 31a to give a DNA chip 31 having the fixed oligonucleotide or polynucleotide 31b.

Separately, a DNA-containing sample 41 is subjected to extraction to separate mRNA or genome DNA 51, from which cDNA or target DNA 52 is obtained. The cDNA or target DNA 52 is labelled with a fluorescence indicator 53a to give a labelled target DNA fragment 53 (which may be a labelled RNA fragment).

The labelled target DNA fragment 53 is then hybridized with the oligonucleotide or polynucleotide 31b of the DNA chip 31, to give a hybridized DNA chip 61. The hybridized DNA chip 61 is scanned by fluorometry in a known DNA scanning fluorometric apparatus, to give a map 71 indicating the positions where the hybridized DNA fragments are present. The known DNA scanning fluorometric apparatus is composed of a fluorescence laser microscope, a chilled CCD camera, and a computer.

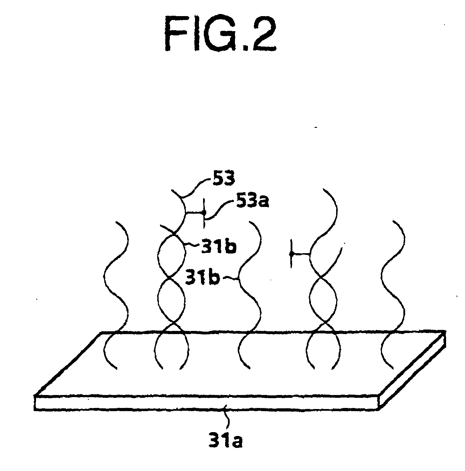

Fig. 2 illustrates an enlarged view of the DNA chip 61 on which fluorescence indicator-labelled complementary DNA fragments are combined to the oligonucleotide by hybridization.

The DNA chip comprises a solid carrier and a great number of oligonucleotides or polynucleotides fixed on the solid carrier.

The solid carrier generally is a sheet of hydrophobic or weak hydrophilic material. For instance, the solid carrier may be a transparent glass sheet, a silicon sheet, or a polymer sheet which is prepared from a polymer such as polyethylene terephthalate, cellulose acetate, polycarbonate of bisphenol A, polystyrene, or poly-(methyl methacryate). A transparent glass sheet and a silicon sheet are preferably employed. More preferably, a transparent glass sheet having a silica coverage is employed. The solid carrier preferably has a thickness of 100 to 2,000 µm.

The solid carrier is preferably pre-treated with a surface-activating agent such as poly-L-lysine, polyethylene imine or polyalkylamine. The poly-L-lysine is most preferred. Otherwise, the glass sheet may be pre-treated with a silane coupling agent having an amino group, an aldehyde group, or an epoxy group. The pretreatment using a silane coupling agent and poly-L-lysine in combination is favorably utilized. The pre-treated solid carrier may be covered on its treated surface with a hydrophilic polymer (which preferably has a positive or negative charge) or a cross-linking agent. The solid carrier *per se* may have a positive or negative charge. The pre-treatment of the solid carrier is favorably employable for enhancing the fixation of oligonucleotide or polynucleotide onto the carrier surface.

Alternatively, or in addition, the oligonucleotide or polynucleotide to be fixed to the solid carrier may be pre-treated, such as for attaching to its terminal group a functional group such as an amino group, an aldehyde group, a thiol group, or a biotin compound. An amino group is preferably employed. These functional groups are effective to enhance electrostatic bonding between the solid carrier and the oligonucleotide or polynucleotide.

The oligonucleotide or polynucleotide can be synthetically prepared, prepared by PCR multiplication method, or prepared by cleaving a single stranded DNA or RNA of natural origin by restriction enzyme. It is preferred that the oligonucleotide or polynucleotide to be fixed onto the solid carrier has a known base sequence.

The oligonucleotide or polynucleotide is dissolved or dispersed in an aqueous solution of a hydrophilic polymer. The aqueous solution containing the oligonucleotide or polynucleotide and a hydrophilic polymer is once placed generally on a plastic plate having 96 or 384 wells, and then spotted onto a solid carrier using a spotting means.

The hydrophilic polymer may be cationic, anionic, or amphoteric. A nonioic polymer is also employable. Preferred is a cationic polymer.

The cationic polymer preferably is a quaternary amine group-containing polymer. Examples of the preferred cationic polymers include poly(1,4-diazoniabicyclo[2.2.2]octane-1,4-diylmethylene-1,4-phenylenemethylene chloride), poly(vinylbenzyltrimethylammonium chloride), poly(methylenetrimethylammonium chloride acrylate), and poly(ethylenetrimethylammonium chloride acrylate). A tertiary amino-group containing polymer such as poly-N-vinylpyrrolidone, polyvinylimidazole, or polyvinylpyrrazole is also preferably employable. Most preferred is poly(1,4-diazoniabicyclo[2.2.2]octane-1,4-diylmethylene-1,4-phenylenemethylene chloride) .

Examples of the nonionic polymers include polyacrylamide, polyethylene glycol, polyvinyl alcohol, acetal derivative of polyvinyl alcohol, cellulose, cellulose derivatives (e.g., hydroxyethylcellulose and hydroxypropylcellulose), and saccharides (e.g., trehalose, sodium alginate, and starch). Preferred are polyacrylamide, polyethylene glycol and trehalose. Most preferred are polyacrylamide and polyethylene glycol.

The anionic polymer preferably has such an anionic group as -COO⁻, -SO₃⁻, -OSO₃⁻, -PO₃⁻, or -PO₂⁻. Preferred anionic polymers are carboxymethylcellulose, cellulose sulfate, polyacrylic acid, polymethacrylic acid, polyvinylbenzenesulfonic acid, or salts of these acid polymers. Most preferred are sodium polyacrylate, sodium polyvinylbenzenesulfonate, and carboxymethylcellulose.

Examples of the amphoteric polymers include proteins such as albumin, gelatin, gelatin derivatives, casein. Albumin is preferred.

The effect of increase of bonding strength formed between the oligonucleotide or polynucleotide and the solid carrier in the presence of a hydrophilic polymer decreases from a cationic polymer (highest), a COO⁻ group-containing anionic polymer, an amphoteric polymer, and a SO₃⁻ group-containing anionic polymer (lowest) in order. The hydrophilic polymer preferably has a molecular weight of 10³ to 10⁶. A hydrophilic polymer having an extremely high molecular weight may produce an extremely high viscosity to give adverse influence to the dissolution of oligonucleotide or polynucleotide in the polymer solution as well as the fixation of the oligonucleotide or polynucleotide onto the solid carrier.

In the solution of oligonucleotide or polynucleotide, the hydrophilic polymer is preferably contained in an amount of 0.1 to 2 vol.%, more preferably in an amount of 0.5 to 1.0 vol.%.

The aqueous solution is spotted onto the solid carrier under the condition that each drop of the solution generally has a volume of 100 pL to 1 µL, preferably 1 to 100 nL. The number of oligonucleotide or polynucleotide is preferably spotted onto the solid carrier in an amount of 10² to 10⁵/cm². In terms of mol., 1 to 10⁻¹⁵ moles are spotted. In terms of weight, several ng or less of oligonucleotide or polynucleotide is spotted. The spotting of the aqueous solution is made onto the solid carrier to form several dots having almost the same form and size. It is important to prepare these dots to have the same form and size, if the hybridization is quantitatively analyzed. Several dots are formed separately from each other with a distance of 1.5 mm or less, preferably 100 to 300 µm. One dot preferably has a diameter of 50 to 300 µm.

After the aqueous solution containing oligonucleotide or polynucleotide and a hydrophilic polymer is spotted onto the solid carrier, the spotted solution is preferably incubated, namely, kept for a certain period at room temperature or under warming, so as to fix the spotted oligonucleotide or polynucleotide onto the carrier. In the course of incubation, UV irradiation or surface treatment using sodium borohydride or a Shiff reagent may be applied. The UV irradiation under heating is preferably adopted. It is assumed that these treatment is effective to produce additional linkage or bridge between the solid carrier and the attached oligonucleotide or polynucleotide. The free (namely, unfixed) oligonucleotide or polynucleotide is washed out with an aqueous solution. The washed solid carrier is then dried to give a DNA chip of the invention.

The DNA chip is favorably employable for monitoring of gene expression, sequencing of base arrangement of DNA, analysis of mutation, analysis of polymorphism, by way of hybridization.

A target DNA fragment or a sample DNA fragment, which is subjected to the analysis concerning the presence of a complementary DNA fragment can be obtained from various origins. In the analysis of gene, the target DNA fragment is prepared from a cell or tissue of eucaryote. In the analysis of genome, the target DNA fragment is obtained from tissues other than erythrocyte. In the analysis of mRNA, the target sample is obtained from tissues in which mRNA is expressed. If the DNA chip has an oligonucleotide fixed in its solid carrier, the target DNA fragment preferably has a low molecular weight. The target DNA may be multiplied by PCR method.

To the target DNA fragment is attached an RI label or a non-RI label by a known method. The non-RI label is preferably utilized. Examples of the non-RI labels include fluorescence label, biotin label, and chemical luminescence label. The fluorescence label is most preferably employed. Examples of the fluorescence labels include cyanine dyes (e.g., Cy3 and Cy5 belonging to Cy Dye™ series), rhodamine 6G reagent, N-acetoxy-N²-acetylaminofluorene (AAF), and AAIF (iodide derivative of AAF). The target or sample DNA fragments labelled with different fluorescence indicators can be simultaneously analyzed, if the fluorescence indicators have fluorescence spectrum of different peaks.

The hybridization is performed by spotting an aqueous sample solution containing a target DNA fragment onto a DNA chip of the invention. The spotting is generally done in an amount of 1 to 100 nL. The hybridization is carried out by keeping the DNA chip having the spotted sample solution thereon at a temperature between room temperature and 70°C, for 6 to 20 hours. After the hybridization is complete, the DNA chip is washed with an aqueous buffer solution containing a surface active agent, to remove a free(unfixed) sample DNA fragment. The surface active agent preferably is sodium dodecylsulfonate (SDS). The buffer solution may be a citrate buffer solution, a phosphate buffer solution, a borate buffer solution, Tris buffer solution, or Goods buffer solution. The citrate buffer solution is preferably employed.

The hybridization on the DNA chip is characteristic in that an extremely small amount of the sample or target DNA fragment is subjected to the analysis. In order to perform the desired hybridization appropriately, optimum conditions should be determined.

The present invention is further described by the following examples.

### Example 1: Fixation of PCR product

### (1) Preparation of slide glass

A slide glass (25 mm x 25 mm) is immersed for one hour in an aqueous ethanolic solution of 50 g of sodium hydroxide in a mixture of 150 mL of distilled water and 200 mL of ethanol. The slide glass is washed with a distilled water and then immersed in an aqueous solution of 10 vol.% of poly-L-lysine (available from Sigma Co.). Thus treated slide glass is centrifuged using a plate centrifuging apparatus, and then dried at room temperature. In the below-mentioned examples, thus treated slide glass was employed.

### (2) Preparation of PCR product (oligonucleotide)

A PCR product prepared from yeast was protected with an amino group at 5'-position and labelled with a fluorescence indicator (FluoroLink Cy5-dCTP, available from Amasham Pharmacia Biotec Corp.).

### (3) Spotting of PCR product-containing solution

An aqueous solution of the above-obtained PCR product in diluted buffer solution (3xSSC, that is, Standard sodium chloride-citrate buffer solution, 0.5 mg/mL) was prepared. To the aqueous PCR product solution was added carboxymethylcellulose (CMC) to give a 1 vol.% CMC solution. The solution containing PCR product and CMC was spotted in an amount of 1 nL onto the glass slide using a spotter. The slide glass was immersed in an aqueous mixture of a standard solution (0.2xSSC) and 0.2 wt.% sodium dodecylsulfate (SDS) solution for 10 minutes under intermittent shaking. The slide glass was then immersed in ethanol and dried at room temperature. Thus dried slide glass was scanned for detecting fluorescence strength. The detected fluorescence strength is set forth in Table 1.

The above-mentioned procedure was repeated using a 0.5 vol.% CMC solution in place of the 1 vol.% CMC solution. The detected fluorescence strength is also set forth in Table 1.

### Comparison Example 1: Fixation of PCR product

The procedures of Example 1 were repeated except that CMC was not added to the solution containing PCR product. The detected fluorescence strength is set forth in Table 1.

### Comparison Example 2: Fixation of PCR product

The procedures of Example 1 were repeated except that sodium hydrogen carbonate was added to the aqueous PCR product solution in place of CMC, to give a 0.35 M sodium hydrogen carbonate solution. The detected fluorescence strength is set forth in Table 1.

### Example 2: Fixation of PCR product

The procedures of Example 1 were repeated except that the slide glass spotted with the solution containing PCR product and CMC (1 vol.% or 0.5 vol.%) was heated in water at 80°C for one hour. The detected fluorescence strength in each run is set forth in Table 1.

### Example 3: Fixation of PCR product

The procedures of Example 1 were repeated except that the slide glass spotted with the solution containing PCR product and CMC (1 vol.% or 0.5 vol.%) was heated in water at 80°C for one hour and then washed with a solution of a mixture consisting 315 mL of 1-methyl-2-pyrrolidone, 5 g of succinic anhydride, and 35 mL of aqueous 1M boric acid solution. The detected fluorescence strength in each run is set forth in Table 1.

### Example 4: Fixation of PCR product

The procedures of Example 1 were repeated except that the slide glass spotted with the solution containing PCR product and CMC (1 vol.% or 0.5 vol.%) was heated in water at 80°C for one hour and then irradiated with ultra-violet(UV) rays at 120 mJ. The detected fluorescence strength in each run is set forth in Table 1.

### Example 5: Fixation of PCR product

The procedures of Example 1 were repeated except that the slide glass spotted with the solution containing PCR product and CMC (1 vol.% or 0.5 vol.%) was heated in water at 80°C for one hour, and thus heated slide glass was irradiated with ultraviolet(UV) rays at 120 mJ, and washed with a solution of a mixture of 315 mL of 1-methyl-2-pyrrolidone, 5 g of succinic anhydride, and 35 mL of aqueous 1M boric acid solution. The detected fluorescence strength in each run is set forth in Table 1.

### Example 6: Fixation of PCR product

The procedures of Example 5 were repeated except that the PCR product prepared from yeast was not protected with an amino group. The detected fluorescence strength in each run is set forth in Table 1.

**Table 1**

| | Additive | Fluorescence strength |
|---|---|---|
| Example 1 | 1 vol.% CMC | 265 |
| | 0.5 vol.% CMC | 159 |
| Com. Ex. 1 | None | 35 |
| Com. Ex. 2 | NaHCO₃ | 26 |
| Example 2 | 1 vol.% CMC | 1233 |
| | 0.5 vol.% CMC | 1254 |
| Example 3 | 1 vol.% CMC | 766 |
| | 0.5 vol.% CMC | 650 |
| Example 4 | 1 vol.% CMC | 1853 |
| | 0.5 vol.% CMC | 1923 |
| Example 5 | 1 vol.% CMC | 1549 |
| | 0.5 vol.% CMC | 1666 |
| Example 6 | 1 vol.% CMC | 353 |
| | 0.5 vol.% CMC | 332 |

The results set forth in Table 1 indicate that the incorporation of carboxymethylcellulose(CMC) into the solution of DNA fragment (i.e., PCR product) is effective to firmly fix the DNA fragment onto the slide glass. Further, activation of DNA fragment by attaching a functional group such as an amino group at its terminal position is effective to enhance the fixation of DNA fragment onto the slide glass. Furthermore, heat treatment or W irradiation enhances the fixation of DNA fragment onto the slide glass.

### Example 7: Detection of complementary DNA fragment

### (1) Preparation of DNA chip

A PCR product was prepared from a gene fragment of yeast (comprising approx. 2,000 base units) and attached with an amino group at its terminal position. The amino group-containing PCR product was dissolved in diluted buffer solution (3 x SSC, that is, Standard sodium chloride-citrate buffer solution, 0.5 mg/mL). To the aqueous PCR product solution was added a hydrophilic polymer (set forth in Table 2) to give a 1 vol.% solution. The solution containing PCR product and hydrophilic polymer was spotted in an amount of 1 nL onto the glass slide using a spotter.

The slide glass spotted with the solution was heated in water at 80°C for one hour, and thus heated slide glass was irradiated with ultraviolet (UV) rays at 120 mJ, and washed with a solution of a mixture of 315 mL of 1-methyl-2-pyrrolidone, 5 g of succinic anhydride, and 35 mL of aqueous 1M boric acid solution.

The slide glass was immersed in an aqueous sodium bromide solution for 10 min. under intermittent shaking. The slide glass was then immersed in ethanol and dried at room temperature, to give a DNA chip of the invention.

### (2) Preparation of labelled DNA fragment

mRNA extracted from yeast was subjected to reverse transcription, and to the produced DNA fragment (cDNA fragment) was attached dCTP having Cy5 label. Thus, a labelled cDNA fragment was obtained.

### (3) Hybridization

The above-obtained cDNA fragment (1 mM) was dispersed in 20 µL of a hybridizing solution (mixture of 4 x SSC and 10 wt.% SDS solution). The cDNA fragment solution was spotted on the DNA chip, and the cDNA fragment solution spotted on the DNA chip was incubated in a moisture chamber at 60°C for 20 hours. The incubated chip was immersed in a mixture of an aqueous 0.1 wt.% SDS solution and a standard solution (2 x SSC). Thus treated chip was washed successively with a mixture of an aqueous 0.1 wt.% SDS solution and a standard solution (2 x SSC), a mixture of an aqueous 0.1 wt.% SDS solution and a standard solution (0.2 x SSC), and a standard solution (0.2 x SSC). The washed chip was centrifuged at 600 r.p.m. for 20 sec., and then dried at room temperature.

The dried slide glass was scanned for detecting fluorescence strength. From the detected fluorescence strength is reduced the background fluorescence strength which was observed when a sample solution containing no fluorescence labelled-DNA fragment was spotted and treated in the same manner. Thus processed fluorescence strength is set forth in Table 2 in terms of a relative value, in which the relative value is expressed in terms of value relative to the fluorescence strength detected on the DNA chip which was treated in the same manner except for using no hydrophilic solution.

**Table 2**

| Hydrophilic polymer | Fluorescence strength |
|---|---|
| None | 1 |
| Poly(1,4-diazoniabicyclo[2.2.2]octane-1,4-diylmethylene-1,4-phenylenemethylene chloride | 20 |
| Polyacrylamide | 10 |
| Polyethylene glycol (M.W.: 4,000) | 10 |
| Polyethylene glycol (M.W.: 20,000) | 2 |
| Polyacrylic acid | 6 |
| Carboxymethylcellulose (CMC) | 5 |
| Albumin | 4 |
| Trehalose | 1.5 |
| Polyvinylbenzenesulfonate | 1.5 |

## Claims

1. A process for preparing a DNA chip comprising a solid carrier and oligonucleotide or polynucleotide which is fixed to the carrier, which comprises spotting an aqueous solution containing the oligonucleotide or polynucleotide and a hydrophilic polymer dissolved therein, so as to fix the oligonucleotide or polynucleotide onto the carrier by the aid of the hydrophilic polymer, washing the spotted carrier, and drying the washed carrier.

2. The process of claim 1, wherein the oligonucleotide or polynucleotide is fixed to the solid carrier at one of its end proportions.

3. The process of claim 1, wherein the solid carrier is a glass sheet, a silicon sheet, or a polymer sheet.

4. The process of claim 1, wherein the solid carrier is a glass sheet which is pre-treated with poly-L-lysine, polyethylene imine or polyalkylamine.

5. The process of claim 1, wherein the solid carrier is a glass sheet which is pre-treated with a silane coupling agent having an amino group, an aldehyde group, or an epoxy group.

6. The process of claim 1, wherein the oligonucleotide or polynucleotide has a functional group selected from the group consisting of an amino group, an aldehyde group, a thiol group, and a biotin group.

7. The process of claim 1, wherein the hydrophilic polymer is a nonionic polymer o a cationic polymer.

8. The process of claim 1, wherein the hydrophilic polymer is a cellulose derivative.

9. The process of claim 1, wherein the hydrophilic polymer is selected from the group consisting of polyacrylamide, polyethylene glycol, polyvinyl alcohol, and saccharide.

10. The process of claim 1, wherein the hydrophilic polymer has a molecular weight of 10³ to 10⁶.

11. The process of claim 1, wherein the aqueous solution containing the hydrophilic polymer in an amount of 0.1 to 2 vol.%.

12. The process of claim 1, wherein the spotted carrier is warmed before the washing.

13. The process of claim 1, wherein the spotted carrier is exposed to UV irradiation before the washing.

## Patentansprüche

1. Verfahren zur Herstellung eines DNA-Chips, umfassend einen festen Träger und ein Oligonukleotid oder Polynukleotid, welches an den Träger fixiert ist, das umfasst Auftüpfeln einer wässrigen Lösung, enthaltend das Oligonukleotid oder Polynukleotid, und ein hydrophiles Polymeres, darin gelöst, zur Fixierung des Oligonukleotids oder des Polynukleotids auf den Träger unter Zuhilfenahme des hydrophilen Polymeren, Waschen des getüpfelten Trägers und Trocknen des gewaschenen Trägers.

2. Verfahren nach Anspruch 1, wobei das Oligonukleotid oder Polynukleotid an den festen Träger an einem seiner Endteile fixiert ist.

3. Verfahren nach Anspruch 1, wobei der feste Träger eine Glasplatte, eine Siliciumfolie oder eine Polymerfolie ist.

4. Verfahren nach Anspruch 1, wobei der feste Träger eine Glasplatte ist, die mit Poly-L-lysin, Polyethylenimin oder Polyalkylamin vorbehandelt wurde.

5. Verfahren nach Anspruch 1, wobei der feste Träger eine Glasplatte ist, die mit einem Silan-Kupplungsmittel, das eine Aminogruppe, eine Aldehydgruppe oder eine Epoxygruppe hat vorbehandelt wurde.

6. Verfahren nach Anspruch 1, wobei das Oligonukleotid oder Polynukleotid eine funktionelle Gruppe aufweist, ausgewählt aus der Gruppe, bestehend aus einer Aminogruppe, einer Aldehydgruppe, einer Thiolgruppe und einer Biotingruppe.

7. Verfahren nach Anspruch 1, wobei das hydrophile Polymere ein nichtionisches Polymeres oder ein kationisches Polymeres ist.

8. Verfahren nach Anspruch 1, wobei das hydrophile Polymere ein Cellulosederivat ist.

9. Verfahren nach Anspruch 1, wobei das hydrophile Polymere ausgewählt wird aus der Gruppe, bestehend aus Polyacrylamid, Polyethylenglykol, Polyvinylalkohol und Saccharid.

10. Verfahren nach Anspruch 1, wobei das hydrophile Polymere ein Molekulargewicht von 10³ bis 10⁶ besitzt.

11. Verfahren nach Anspruch 1, wobei die wässrige Lösung das hydrophile Polymere in einer Menge von 0,1 bis 2 Vol.-% enthält.

12. Verfahren nach Anspruch 1, wobei der getüpfelte Träger vor dem Waschen erwärmt wird.

13. Verfahren nach Anspruch 1, wobei der getüpfelte Träger vor dem Waschen UV-Bestrahlung unterworfen wird.

## Revendications

1. Procédé de préparation d'une puce à ADN comprenant un support solide et un oligonucléotide ou un polynucléotide qui est fixé au support, qui comprend le dépôt d'une solution aqueuse contenant l'oligonucléotide ou le polynucléotide et un polymère hydrophile dissous dans celle-ci, de manière à fixer l'oligonucléotide ou le polynucléotide sur le support à l'aide du polymère hydrophile, le lavage du support ayant subi le dépôt et le séchage du support lavé.

2. Procédé selon la revendication 1 où l'oligonucléotide ou le polynucléotide est fixé au support solide à l'une de ses parties terminales.

3. Procédé selon la revendication 1 où le support solide est une feuille de verre, une feuille de silicium ou une feuille de polymère.

4. Procédé selon la revendication 1 où le support solide est une feuille de verre qui est prétraitée avec la poly-L-lysine, la polyéthylène imine ou une polyalkylamine.

5. Procédé selon la revendication 1 où le support solide est une feuille de verre qui est prétraitée avec un agent de couplage silane ayant un groupe amino, un groupe aldéhyde ou un groupe époxy.

6. Procédé selon la revendication 1 où l'oligonucléotide ou le polynucléotide a un groupe fonctionnel choisi dans le groupe consistant en un groupe amino, un groupe aldéhyde, un groupe thiol et un groupe biotine.

7. Procédé selon la revendication 1 où le polymère hydrophile est un polymère non ionique ou un polymère cationique.

8. Procédé selon la revendication 1 où le polymère hydrophile est un dérivé cellulosique.

9. Procédé selon la revendication 1 où le polymère hydrophile est choisi dans le groupe consistant en le polyacrylamide, le polyéthylène-glycol, le poly(alcool vinylique) et un saccharide.

10. Procédé selon la revendication 1 où le polymère hydrophile a une masse moléculaire de 10³ à 10⁶.

11. Procédé selon la revendication 1 où la solution aqueuse contient le polymère hydrophile en une quantité de 0,1 à 2 vol.%.

12. Procédé selon la revendication 1 où le support ayant subi le dépôt est chauffé avant le lavage.

13. Procédé selon la revendication 1 où le support ayant subi le dépôt est exposé à une irradiation UV avant le lavage.
